Europäisches Patentamt

European Patent Office

Office européen des brevets

(11) Publication number: **0 172 680 B1**

# EUROPEAN PATENT SPECIFICATION

(45) Date of publication of patent specification: **25.11.92**   (51) Int. Cl.⁵: **A61B 1/06**, A61B 1/04

(21) Application number: **85305353.6**

(22) Date of filing: **26.07.85**

(54) **Sequential colour light sources for endoscopes of the type employing a solid-state imaging device.**

(30) Priority: **31.07.84 JP 162522/84**

(43) Date of publication of application:
**26.02.86 Bulletin  86/09**

(45) Publication of the grant of the patent:
**25.11.92 Bulletin  92/48**

(84) Designated Contracting States:
**AT BE CH DE FR GB IT LI NL SE**

(56) References cited:
EP-A- 0 018 874        EP-A- 0 048 410
EP-A- 0 050 834        EP-A- 0 115 874
GB-A- 1 340 767        JP-U- 5 740 408
US-A- 4 074 306

H. Baumann, G. Schröder: Bauelemente der
Optik, (München, Wien), pp 260,261

(73) Proprietor: **OLYMPUS OPTICAL CO., LTD.**
**43-2, 2-chome, Hatagaya Shibuya-ku**
**Tokyo 151(JP)**

(72) Inventor: **Fujimori, Hiroyoshi**
**Olympus Ishikawa ryo 2544, Ishikawa-cho**
**Hachioji-shi Tokyo(JP)**
Inventor: **Nagasaki, Tatsuo**
**3-24-3, Kyonan-cho**
**Musashino-shi Tokyo(JP)**
Inventor: **Kato, Tadashi**
**Olympus Higashi Asakawa ryo 11-1, Higashi**
**Asakawa-machi Hachioji-shi Tokyo(JP)**
Inventor: **Sasaki, Masahiko**
**Olympus Higashi Asakawa ryo 11-1, Higashi**
**Asakawa-machi Hachioji-shi Tokyo(JP)**

(74) Representative: **Bridge-Butler, Alan James et**
**al**
**G.F. REDFERN & CO. High Holborn House**
**52/54 High Holborn**
**London WC1V 6RL(GB)**

EP 0 172 680 B1

## Description

This invention relates to sequential colour light sources employing interference filters, for use with endoscopes of the type employing a solid-state imaging device.

Recently, optical pick-up transducers employing a solid-state imaging device (SID) have become popular for television cameras, endoscopes, and the like.

Existing endoscopes employ as a light guide for illuminating light transmitting means a very compact bundle containing a large number, say several tens of thousands, of optical fibres, each having a diameter of several tens of microns. For image-transmitting means an optical system may be used, or an image guide of such optical fibres. An endoscope is designed so that an image of a subject is projected by a focussing optical system at the distal tip of an endoscope insertion sheath, to be focussed on the input face of the image guide, if present, and the image projected through said image guide fed into a passage in the handle grip member at the trailing end of said endoscope image guide so as to be observed through an eyepiece optical system.

In such an endoscope employing said solid-state imaging device in order to diagnose correctly, a coloured image is a great aid to observation.

The most popular means for picking up a coloured image employ a combination of separate colour optical transducers, possibly using many monochrome solid-state imaging devices with respective red, green and blue filters in a mosaic arrangement.

In this former type, however, it is difficult to incorporate a colour separation optical system and three suitable solid-state imaging devices in a thin and small space such as the insertion sheath of an endoscope. If a large number of transducers are used, since the colour components feed the imaging devices separately, one third of the solid-state imaging devices should be allotted to each colour component, so that the resolution is reduced as compared with monochrome images.

In addition in the latter type, since the imaging devices for receiving each colour component differ, colour registration frequently cannot be achieved by mixing the colour components. For such reasons, prior art proposals have provided illuminating means with filters for sequentially illuminating the subject with each primary colour successively, as will be shown hereinafter.

In such cases, however, if absorption filters are employed in a rotary filter, the low thermal stability of such filters leads to a tendency to degrade the image very quickly. If highly stable, heat-resistant interference filters are employed, but the light is incident on said filters over a large range of incident angles, the transmitted light is shifted and reproducibility of colour degraded to such an extent that the coloured display of a subject cannot be achieved with sufficient fidelity, and this causes serious trouble in diagnosis. Prior art as disclosed, for example, in the Japanese Utility Model Application No. 114405/1980 laid open to public inspection under Publication No. 40408/1982 has such disadvantages.

One object of the present invention is to provide a light source that offers high colour reproducibility for an endoscope of the type employing a solid-state imaging device, for low cost, but optimum illuminating light intensity.

According to the present invention there is provided a sequential colour light source for an endoscope incorporating an elongate insertion sheath housing at its distal end an objective lens with a solid-state imaging device aligned with the focal plane of said objective lens, and a light guide passing through said insertion sheath to project illuminating light into a subject positioned before said objective lens, said light source comprising a lamp, a reflector producing a substantially parallel light beam, a converging convex lens system concentrating the light beam onto the input end of an output light guide, and a rotary colour filter wheel formed with independent colour-transmitting filters positioned in the optical path from said light source to the input end of said light guide to sequentially illuminate a subject with light having different respective wavelengths, characterised in that the colour-transmitting filters are interference filters, and in that two lens systems and the filter wheel are arranged in the optical path, the first of said two lens systems being located in said path between the light source and the filter wheel, and the second of said lens systems being located in said path between the filter wheel and the input end of the light guide, wherein said first lens system reduces the cross-sectional area of the beam perpendicular to the optical axis and either makes the beam parallel, or convergent at an acute angle of incidence, when it is incident on one of the interference filters, and wherein said second lens system makes the beam convergent at a larger angle of incidence compared with the acute angle of incidence when it is incident on the input end of the light guide.

The invention will now be described with reference to the drawings, in which:-

Figure 1 is a schematic representation of the optical system of one known light source embodiment;

Figure 2 is a schematic representation of the optical system of another known light source embodiment;

Figure 3 is a block schematic diagram showing a first exemplary embodiment of the invention, including fragmentary details illustrating the structure of an endoscope and display system fitted to said first embodiment;

Figure 4 is a front view of a rotary filter wheel used in the embodiment illustrated in Figure 3;

Figure 5 is a schematic view illustrating a second exemplary embodiment of the present invention;

Figure 6 is a schematic view of modification of said second exemplary embodiment constructed in accordance with this invention;

Figure 7 is a schematic view of the light source optical system of a third exemplary embodiment constructed in accordance with the present invention; and

Figure 8 is a schematic view of a fourth exemplary embodiment constructed in accordance with the present invention.

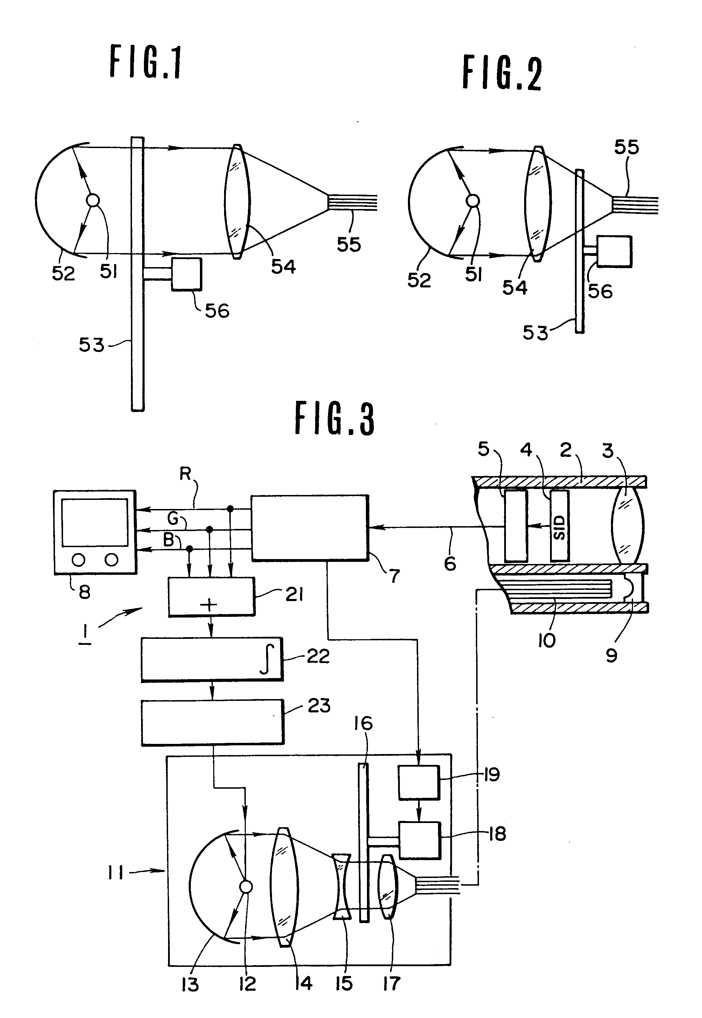

In the known embodiment shown in Figure 1, light from a light source 51 is reflected by a concave mirror 52 to form a substantially parallel beam of light, which is filtered by a rotary colour filter wheel 53 mounted on the beam-path to a condensor lens 54 which forms a convergent beam to concentrate the light on an input face of a light guide 55. The subject to be viewed is illuminated by the light issued from the output end of the light guide 55, possibly after being coupled in to a further light guide within the insertion sheath of an endoscope (not shown).

The rotary filter wheel 53 comprises a circular disk divided into three sectors, each containing a different colour filter, and it is rotated by a driving motor 56, so that respective electrical signals for each image are picked up by the sequential illuminating in each colour and can be combined to be displayed as full colour images.

In the above-mentioned prior art, absorption filters or heat-resistant interference filters may be employed in the rotary filter wheel 53, but the relatively large size of the rotary filter wheel requires a design of high mechanical strength, and a large capacity driving motor 56, thereby increasing the costs, enlarging the structures and increasing the weight.

On the other hand, the filter area illuminated by light can be reduced and the configuration, i.e. the total area of the rotary filter wheel 53 can be reduced by inserting the rotary filter wheel 53 in the beam path at a point having a small area, between the condensor lens 54 and the input face of the light guide 55, as shown in the known arrangement represented in Figure 2, so that the filter can be driven by a smaller driving motor 56 requiring less torque.

A first exemplary embodiment constructed in accordance with the present invention is illustrated in Figure 3, for use with an endoscope 1 provided with a focussing lens 3 at the distal tip of an insertion sheath 2, a solid-state imaging device 4 such as CCD (charge-coupled device) or the like being positioned in the focal plane of said objective lens 3. A great number of photo-electric transducer elements are arranged in a regular array in the pick-up plane of said solid-state imaging device 4. Each transducer receives a video picture image element, and issues a photo-electrically converted electrical signal corresponding to each picture element, which are read successively by clock signals from a generator (not shown). The video signal thus read-out is amplified in a preamplifier 5 having a low noise factor, and is then fed to a video processor 7 via a signal cable 6. Said video processor 7 converts the analogue input signals into digital form, and each monochromatic image provided by the sequential illumination with monochromatic light is changed over through a multiplexer to be memorized into each exclusive frame memory, as will be referred to later. During the reading mode, the memorized signals (data) are read, converted by a D/A converter into analogue colour signals R, G and B to be amplified and added, with horizontal and vertical synchronising signals (not shown) and sent to a colour television monitor 8 to be displayed as a colour image.

Within said insertion sheath 2, a light-distributor lens 9 is incorporated in close relationship to the objective lens 3, and projects light from the light guide 10 which has been inserted into the insertion sheath 2 so that the output face thereof is positioned closely behind said distributor lens 9.

At the handle grip end of the insertion sheath, an input face of said light guide 10 is detachably coupled to receive incident light fed from the light source 11 via a linking light guide, as was described with reference to Figure 1.

Within said light source 11, illuminating light from a lamp 12 is reflected by a reflector 13 having a concave or parabolic surface, to form a substantially parallel beam of light, and this light beam is rendered convergent by a first convex lens system 14, and is then incident upon a concave lens system 15 having a smaller aperture, to be issued as a substantially parallel beam of light having a reduced cross-section. This light beam is passed through a rotary filter wheel 16, to be rendered convergent again by a second convex lens system 17, and directed to the end face of the light guide 10, which lies in the vicinity of its focus. The rotary filter wheel 16 is installed on the optical path between said concave lens system 15 and said convex lens system 17, say, at the pupil of the latter lens system 17. The illuminating light is incident on the input face of the light guide at the predetermined maximum angle of incidence, and is transmitted through the core of optical fibres, while being totally internally reflected by the boundary surface between said core and a peripherally-clad layer, to be projected on to the subject that is to be observed, directly or after being rendered divergent by the distributor lens 9. The use of such a divergent illuminating light can give a substantially uniformly illumination over the range that can be focussed through the objective lens 3.

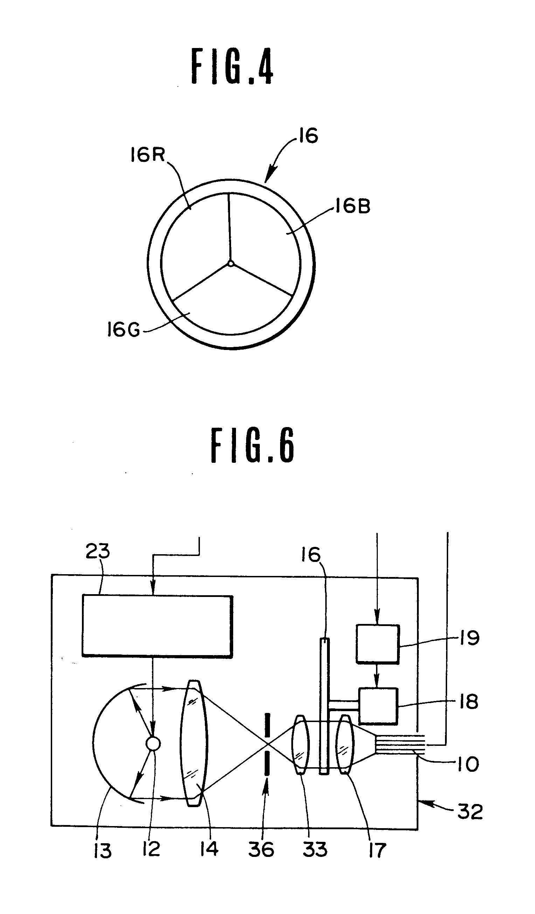

As shown in Figure 4, said rotary filter wheel is composed of a red transmitting filter 16R, a green transmitting filter 16G and a blue transmitting filter 16B, each transmitting exclusively light having a red, green or blue wavelength, and each in the form of sector including an angle of 120°, the filter wheel 16 being rotated by a motor 18.

The motor 18 is driven by the power supplied through a motor driving circuit 19, and it may be, for example, a pulse-stepping motor rotating by a predetermined angle with each input pulse, and designed so that the driving pulse is not applied to the motor 18 for the predetermined short time during which each colour filter, 16R, 16G or 16B, is positioned on the optical path between the concave lens 15 and the convex lens 17, and then applied to the motor after expiration of such a predetermined time to shift the filter wheel quickly to bring in the subsequent colour filter for the sequential colour illumination, in order to illuminate the subject sequentially with three colours through the three colour filters 16R, 16G and 16B.

Each driving pulse for said motor driving circuit 19 is issued by the controlling signal supplied from the video processor 7.

The colour filters 16R, 16G and 16B for said rotary filter wheel 16 are formed as interference filters (vacuum deposited film filters) each prepared by laminating a number of transparent dielectric films of a thickness depending on its purpose, onto a glass substrate by vacuum deposition or the like, so as to pass exclusively light having a given wavelength, utilizing the interference effect of light passing through such films. Since such an interference filter has high thermal stability and heat-resistance, it can be employed in zones having high light density such as the converged parallel light beam in this first described embodiment.

In addition, this first embodiment is provided with automatic light-controlling means for preventing any shortcoming when a subject positioned at a short distance receives excessive illuminating light, or if high-light portions having high reflection are present on a subject, when the excessive light reflection may form blooming or a whitish tone, preventing adequate contrast, and similar shortcomings.

The colour signals R, G, B issued from the video processor 7 are added in an adder 21 to form brightness signal components, which are integrated in an integrating circuit 22 having a time constant of about one frame period, to be applied to a control terminal of a light source driver 23 provided for the lamp 12 and act as a light-controlling signal to control the luminous intensity of the lamp 12, i.e. the illuminating intensity projected from the tip of the light guide 10.

The light source driver 23 can employ a power amplifier circuit or the like, which variably controls the output current or output voltage so that output is reduced as the bias level applied to the control terminal of driver 23 increases.

The functioning of this first embodiment having the above-described construction is as follows. When the distal tip of the insertion sheath 2 of the endoscope 1 is brought to the vicinity of a subject such as diseased organ, or held at a distance from the subject to permit general diagnosis of an overall symptom, the light intensity incident from the illuminated subject is varied in accordance with the distance to the subject, so that the optimum illuminating intensity is obtained. The signals corresponding to each picture element and issued from the solid-state imaging device 4 under such conditions are fed into the video processor 7 and memorized in each frame memory for one frame for each colour. When the subject is illuminated and the three colours recorded sequentially the data in each frame memory can be read out concurrently, D/A converted to form colour signals R, G and B, and displayed on a colour television monitor 8 as a coloured image, and in addition to being fed into adder 21 to form automatic light-control means.

The signals are converted into a brightness signal by the adder 21, to form a light intensity control signal in the integrating circuit 22, controlling the output from the light source driver 23, depending on the level of said control signal. In short, if the illuminating light intensity is excessively high, increasing the level of the control signal, then the output from the light source driver 23 is reduced, and if the illuminating light intensity is excessively low, decreasing the level of the control signal, the illuminating intensity from the lamp 12 is controlled to an appropriate value during the period of one frame from one colour frame to subsequent colour frame (i.e. the period for 3 frames for each colour). Accordingly, the operators time is freely available, without having to adjust the illuminating light strength, and can be devoted conveniently to the diagnosis or medical treatment.

Furthermore in the first embodiment, the sequential colour illuminating means comprise converging the illuminating light through a first convex lens system 14, forming a parallel light beam having a smaller cross-section in the concave lens system 15, and passing this parallel light beam through the rotary filter wheel 16, comprising heat-resistant interference filters, so that the means can prevent an incomplete illuminating light spectrum, such as might be obtained in the prior art. The rotary filter wheel 16 having a smaller size can be employed, thereby reducing the torque required for driving said rotary filter wheel 16 and enabling a smaller motor 17 to be used. Accordingly, the entire light source construction 11 can be miniaturized and cost reduced. Furthermore, since the means provide sequential colour illumination, subjects can be picked up and displayed clearly to fine limits without degrading the resolving power, thereby contributing markedly to the diagnosis efficiency.

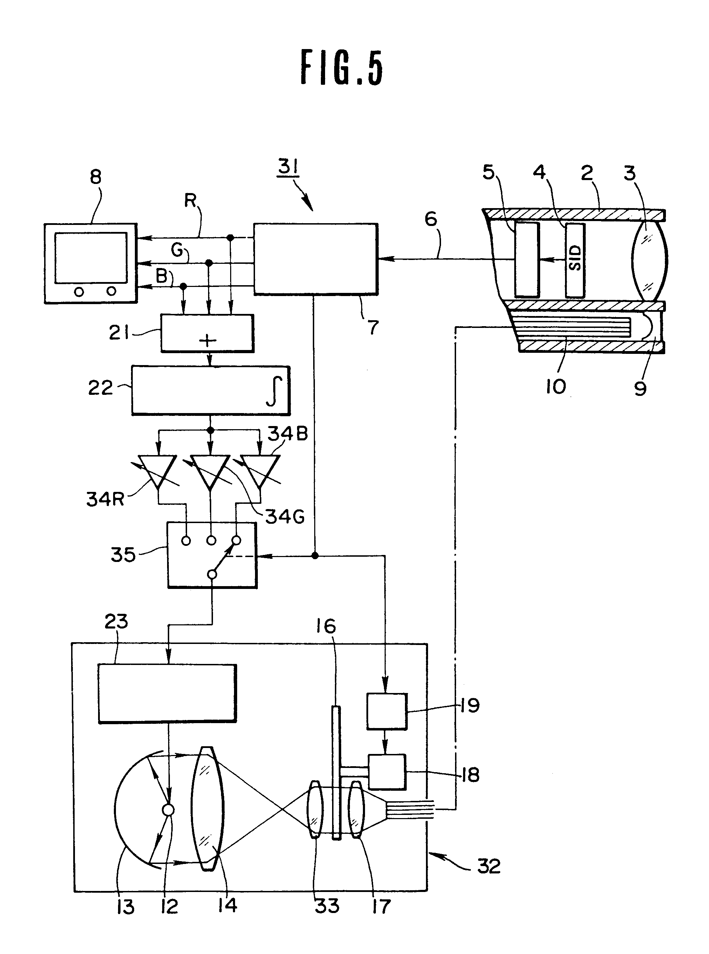

A second exemplary embodiment according to this invention is shown in Figure 5, which illustrates an endoscope 31 together with a light source 32, in which a convex lens system 33 is used in lieu of the concave lens system 15 used in said first embodiment.

In the second embodiment, a parallel light beam reflected by the reflector 13 is rendered convergent by a first convex lens system 14, and focussed to a point in the focal plane, when it then diverges and passes through a convex lens system 33 arranged on the divergent optical path to provide a substantially parallel light beam of reduced cross-section. In short, said convex lens system 33 is arranged on the optical axis beyond the focal plane. The rotary filter wheel 16 is interposed between said convex lens system 33 and a second convex lens system 17, which is provided for converging the parallel light beam in a similar manner to that described for the first embodiment.

On the other hand, automatic light intensity control means are provided with colour-complemental means. The output terminal of the integrating circuit 22 is connected to each input terminal of colour-complemental preset amplifiers 34R, 34G and 34B, and each output terminal is connected to the control terminal of light source driver 23 via a multiplexer 35.

Said multiplexer 35 is controlled in synchronisation with the rotary filter wheel 16 and connects sequentially each preset amplifier 34R, 34G or 34B to the light source driver 23 in synchronisation with the illumination through each colour-transmitting filter, 16R, 16G or 16B.

Said preset amplifiers 34R, 34G and 34B act to correct the intensity distribution of the illuminating light spectrum issued from the lamp 12, allowing for the transmitting characteristics for different wavelengths in the light guide 10, and the photosensitive characteristics of the solid-state imaging device 4.

The second exemplary embodiment functions otherwise in a similar manner to the first embodiment.

The effects achieved by said second embodiment are substantially equal to those achieved by said first embodiments. The additional provision of colour-complementary means can control automatically the illuminating light by correcting colours independently, so that the subject can be displayed in colour with higher fidelity.

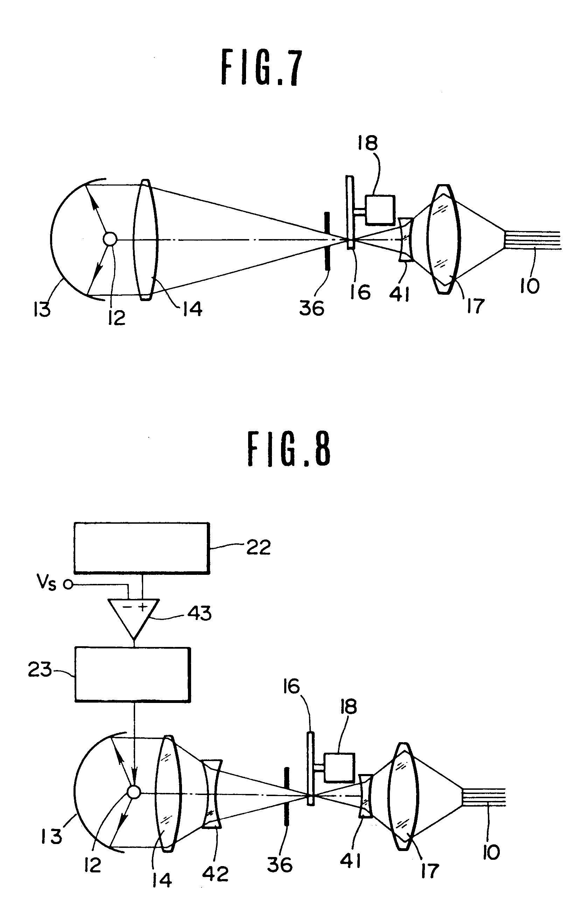

A modification of this second exemplary embodiment is illustrated in Figure 6, which has an iris 36 provided at the focus of the first converging convex lens system 11, to block harmful light which cannot be deflected to a parallel light beam through the convex lens system 33. In short, the first convex lens system 14 has a larger aperture, so as to provide easily and economically an optical system with low aberration. Moreover, it is otherwise expensive to deflect the light to form a completely parallel light beam and the size of light source lamp 12 is restricted. For such reasons, the light passed through the convex lens system 14 cannot always be focussed in the focal plane. The iris 36 removes any light component comparatively remote from said point, and the substantially focussed light is incident to convex lens system 33 for providing a substantially parallel light beam.

As said convex lens system 33 has a smaller aperture, the system with relatively low aberration can be realised inexpensively, and the light passed through said convex lens system 33 turned to a substantially parallel light beam. Consequently, when interference filters are employed in the rotary filter wheel 16, the incident angle is substantially 0° so as to prevent any shift of the transmitted light from the normal wavelength and to provide illuminating means with reliable colour reproducibility.

Figure 7 illustrates details of an optical system used in a light source constructed in accordance with a third exemplary embodiment of this invention.

In this optical system, in the light source a first convex lens system 14 has a long focal distance, an iris 36 removes harmful light midway, and a rotary filter wheel 16 is installed at the vicinity of focus of said convex lens system 14.

Thanks to the convex lens system 14 having a long focal distance, the maximum angle of incidence on the rotary filter wheel is predetermined within a range minimising the shift of wavelength, for example, within a value of lower than 15°, and by predetermining the maximum incident angle to less than 15°, the median wavelength is not significantly shifted. (For example, if the median wavelength of light incident at an angle of 0° and filtered through the filter is assumed to be 1, then the median wavelength of light incident at an angle of 15° is shifted from 1 to 0.99).

After passing through said rotary filter wheel 16, the light is diverged by the concave lens system 41 and then converged by the converging convex lens system 17 to be incident on the end face of the light guide at a larger incident angle and to issue from the distal end face of light guide 10 as divergent illuminating light.

Said third embodiment operates with substantially equal effect to said first embodiment.

In addition, the curvature of the convex lens system can be reduced by employing lens system having long focal distance so that the aberration can be reduced. Moreover, interference filters having very small area can be employed by positioning the rotary filter wheel at the focus or in the vicinity of focus of the convex lens system 14.

Figure 8 illustrates a fourth exemplary embodiment of this invention, designed to modify the optical system shown in Figure 7 by reducing the focal distance of the converging convex lens system 14 to a convergent light beam on a longer path, by providing a concave lens system 42 to render the convergent light beam from the convex lens system 14 less convergent in the lens system 42, thereby reducing sufficiently the maximum incident angle at the rotary filter wheel 16, as in said third embodiment. The length of the optical system can be reduced or the outer shape of the optical system can be minimized by interposing a concave lens system 42 in lieu of the directly incident light beam converged through the convex lens system 14 and iris 36.

On the other hand, in this embodiment, the signal passed through the integrating circuit 22 is applied to a non-inverting input terminal of a comparator 43 and the other input terminal has a reference voltage Vs applied.

Accordingly, if the control signal passed through integrating circuit 22 exceeds the predetermined reference voltage Vs, the output from comparator 43 is reversed or high level to nullify the driving current or voltage for light source driver 23 and to extinguish the lamp 12.

In short, this embodiment provides illuminating light having a constant intensity but optimizes the dosage by controlling the illuminating time.

This embodiment is suitable when the intensity of emission spectrum is easily varied by any change in the supplied current or the like, as in the lamp 12.

A luminous diode or the like may be employed in lieu of a lamp 12.

## Claims

1. A sequential colour light source for an endoscope (1) incorporating an elongate insertion sheath (2) housing at its distal end an objective lens (3) with a solid-state imaging device (4) aligned with the focal plane of said objective lens, and a light guide (10) passing through said insertion sheath to project illuminating light into a subject positioned before said objective lens, said light source comprising a lamp (12), a reflector (13) producing a substantially parallel light beam, a converging convex lens system concentrating the light beam onto the input end of an output light guide, and a rotary colour filter wheel (16) formed with independent colour-transmitting filters (16B, 16G, 16R) positioned in the optical path from said light source to the input end of said light guide to sequentially illuminate a subject with light having different respective wavelengths, characterised in that the colour-transmitting filters are interference filters, and in that two lens systems and the filter wheel (16) are arranged in the optical path, the first (14,15; 14,33; 14,33,36; 14,36; 14,36,42) of said two lens systems being located in said path between the light source and the filter wheel, and the second (17; 41,17) of said lens systems being located in said path between the filter wheel and the input end of the light guide, wherein said first lens system reduces the cross-sectional area of the beam perpendicular to the optical axis and either makes the beam parallel (Figs. 3,5,6), or convergent at an acute angle of incidence (Figs. 7,8), when it is incident on one of the interference filters, and wherein said second lens system makes the beam convergent at a larger angle of incidence compared with the acute angle of incidence when it is incident on the input end of the light guide.

2. A source as claimed in claim 1, characterised in that said first lens system comprises a convex lens (14) and a concave lens (15;42).

3. A source as claimed in claim 1, characterised in that said first lens system comprises two convex lenses (14,33).

4. A source as claimed in claim 1, characterised in that said first lens means is a single convex lens (14).

5. A source as claimed in claim 3 or claim 4, characterised in that an iris (36) is provided at the vicinity of a focus of the converging light beam before said filter wheel.

6. A source as claimed in anyone of the preceding claims, characterised in that said second lens system is a single convex lens (17).

7. A source as claimed in any one of claims 1 to 5, characterised in that said second lens system comprises a concave lens (41) and a convex lens (17).

8. An endoscope comprising a light source as claimed in any of the preceding claims, characterised in that said solid-state imaging device (4) feeds an output signal to a video processor (7) for memorising the electrical signals corresponding to the picture elements issued from said solid-state imaging device, defined by applying clock signals to cause sequential colour signals to be issued to display means (8) for displaying colour signals fed from said video processor.

9. An endoscope as claimed in claim 8, characterised in that means (21) are provided for automatically controlling the light intensity for forming a brightness signal by adding the colour signals from said endoscope, means (22) for integrating said brightness signals for a time constant of approximately one frame period, and means (23) for controlling the intensity of illuminating light from said light source by applying the output from said integrating means to the power source for the lamp of said light source.

**Patentansprüche**

1. Sequentielle Farblichtquelle für ein Endoskop (1), das eine längliche Einsatzhülle (2) umfaßt, die an ihrem distalen Ende eine Objektivlinse (3) mit einem Festkörperbildaufnahmeelement in Flucht mit der Fokalebene dieser Objektivlinse umschließt, und einem durch die Einsatzhülle hindurchgehenden Lichtleiter (10), um Beleuchtungslicht auf ein vor dieser Objektivlinse angeordnetes Objekt zu werfen, diese Lichtquelle eine Lampe (12), einen Reflektor (13) zur Erzeugung eines im wesentlichen parallelen Lichtstrahls, ein konvergierendes konvexes Linsensystem zur Konzentration des Lichtstrahls auf das Eingangsende eines Ausgangslichtleiters und ein rotierendes Farbfilterrad (16) aufweist, das aus unabhängigen farbdurchlässigen Filtern (16B, 16G, 16R) besteht und im Strahlengang von der Lichtquelle und dem Eingangsende des Lichtleiters zur sequentiellen Beleuchtung eines Objekts mit Licht verschiedener entsprechender Wellenlängen angeordnet ist, **dadurch gekennzeichnet**, daß die farbdurchlässigen Filter Interferenzfilter sind und daß zwei Linsensysteme und das Filterrad (16) im Strahlengang angeordnet sind, das erste (14, 15; 14, 33; 14, 33, 36; 14, 36; 14, 36, 42) dieser beiden Linsensysteme in diesem Strahlengang zwischen der Lichtquelle und dem Filterrad angeordnet ist, und das zweite (17; 41, 17) dieser Linsensysteme in diesem Strahlengang zwischen dem Filterrad und dem Eingangsende des Lichtleiters angeordnet ist, wobei das erste Linsensystem die Querschnittsfläche des Strahls rechtwinklig zur optischen Achse reduziert entweder durch paralleles Ausrichten des Strahls (Figuren 3, 5, 6) oder durch Konvergieren auf einen spitzen Einfallswinkel (Figuren 7, 8), wenn er auf einem der Interferenzfilter auftrifft, und wobei das zweite Linsensystem den Strahl konvergent auf einen größeren Einfallswinkel verglichen mit dem Einfallswinkel, wenn er auf das Einfallsende des Lichtleiters auftrifft, ausrichtet.

2. Quelle nach Anspruch 1, **dadurch gekennzeichnet**, daß das erste Linsensystem eine konvexe Linse (14) und eine konkave Linse (15; 42) umfaßt.

3. Quelle nach Anspruch 1, **dadurch gekennzeichnet**, daß das erste Linsensystem zwei konvexe Linsen (14, 33) umfaßt.

4. Quelle nach Anspruch 1, **dadurch gekennzeichnet**, daß das erste Linsensystem eine einzige konvexe Linse (14) ist.

5. Quelle nach Anspruch 3 oder 4, **dadurch gekennzeichnet**, daß in der Nähe eines Brennpunkts des konvergierenden Lichtstrahls vor dem Filterrad eine Irisblende (36) angeordnet ist.

6. Quelle nach einem der vorhergehenden Ansprüche, **dadurch gekennzeichnet**, daß das zweite Linsensystem eine einzige konvexe Linse (17) ist.

7. Quelle nach einem der Ansprüche 1 bis 5, **dadurch gekennzeichnet**, daß das zweite Linsensystem eine konkave Linse (41) und eine konvexe Linse (17) umfaßt.

8. Endoskop mit einer Lichtquelle nach einem der vorhergehenden Ansprüche, **dadurch gekennzeichnet**, daß das Festkörperbildelement (4) ein Ausgangssignal einem Videoprozessor (7) zuführt zur Speicherung der elektrischen Signale entsprechend den vom Festkörperbildaufnahmeelement erzeugten Bildelemente definiert durch Anlegen von Taktsignalen zur Veranlassung der Ausgabe sequentieller Farbsignale an Anzeigemittel (8) zur Anzeige von vom Videoprozessor zugeführten Farbsignalen.

9. Endoskop nach Anspruch 8, **dadurch gekennzeichnet**, daß Mittel (21) zur automatischen Steuerung der Lichtintensität zur Bildung eines Helligkeitssignals vorgesehen sind durch Addieren der Farbsignale vom Endoskop, Mittel (22) zum Integrieren dieser Helligkeitssignale für eine Zeitkonstante näherungsweise einer Bildperiode und Mittel (23) zur Steuerung der Intensität des Beleuchtungslichts von dieser Lichtquelle vorgesehen sind durch Anlegen des Ausgangs dieser Integrationsmittel an die Stromquelle für die Lampe dieser Lichtquelle.

**Revendications**

1. Source lumineuse chromatique séquentielle pour un endoscope (1) comportant une gaine d'introduction allongée (2) comprenant à son extrémité distale un objectif (3) avec un dispositif de formation d'image à l'état solide (4) aligné sur le plan focal dudit objectif, et un guide de lumière (10) traversant ladite gaine d'introduction pour projeter une lumière d'éclairement sur un objet disposé devant ledit objectif, ladite source lumineuse comportant une lampe (12), un réflecteur (13) produisant un faisceau lumineux sensiblement parallèle, un système de lentilles convexes convergent concentrant le faisceau lumineux sur l'extrémité d'entrée d'un guide de lumière de sortie, et une roue de filtre de couleur rotative (16),constituée de filtres de transmission de couleur indépendants (16B, 16G,16R),positionnée dans le trajet optique depuis ladite source lumineuse vers l'extrémité d'entrée dudit guide de lumière pour éclairer séquentiellement un objet avec une lumière ayant des longueurs d'onde respectives différentes,
caractérisée en ce que les filtres de transmission de couleur sont des filtres d'interférence, et en ce que deux systèmes de lentilles et la roue de filtre (16) sont disposés sur le trajet optique, le premier (14,15;14,33;14,33,36;14,36;14,36, 42) desdits deux systèmes de lentilles étant disposé sur ledit trajet entre la source lumineuse et la roue de filtre,et le second (17;41,17) desdits systèmes de lentilles étant disposé sur ledit trajet entre la roue de filtre et l'extrémité d'entrée du guide de lumière, dans lequel ledit premier système de lentilles réduit la surface en coupe du faisceau perpendiculaire à l'axe optique et, soit rend le faisceau parallèle (Figs.3,5,6), soit convergent selon un angle aigu d'incidence (Figs.7,8), lorsqu'il frappe l'un des filtres d'interférence, et dans lequel ledit second système de lentilles rend le faisceau convergent selon un angle d'incidence plus grand en comparaison de l'angle aigu d'incidence lorsqu'il frappe l'extrémité d'entrée du guide de lumière.

2. Source selon la revendication 1, caractérisée en ce que ledit premier système de lentilles comporte une lentille convexe (14) et une lentille concave (15;42).

3. Source selon la revendication 1, caractérisée en ce que ledit premier système de lentilles comporte deux lentilles convexes (14,33).

4. Source selon la revendication 1, caractérisée en ce que ledit premier système de lentilles comportent une unique lentille convexe (14).

5. Source selon la revendication 3 ou la revendication 4, caractérisée en ce qu'un iris (36) est disposé à proximité d'un foyer du faisceau de lumière convergent devant ladite roue de filtre.

6. Source selon l'une quelconque des revendications précédentes, caractérisée en ce que ledit second système de lentilles comporte une unique lentille convexe (17).

7. Source selon l'une quelconque des revendications 1 à 5, caractérisée en ce que ledit second système de lentilles comporte une lentille concave (41) et une lentille convexe (17).

8. Endoscope comportant une source lumineuse selon l'une quelconque des revendications précédentes, caractérisé en ce que ledit dispositif de formation d'image à l'état solide (4) délivre un signal de sortie à un vidéoprocesseur (7) pour mémoriser les signaux électriques correspondants aux éléments d'image délivrés par ledit dispositif de formation d'image à l'état solide, définis en appliquant des signaux d'horloge pour provoquer la délivrance de signaux de couleur séquentiels à des moyens d'affichage (8) pour afficher des signaux de couleur délivrés par ledit vidéoprocesseur.

9. Endoscope selon la revendication 8, caractérisé en ce que des moyens (21) sont prévus pour commander automatiquement l'intensité lumineuse pour former un signal de luminosité par addition des signaux de couleur provenant dudit endoscope, des moyens (22) pour intégrer lesdits signaux de luminosité pour une constante de temps d'environ une période de trame, et des moyens (23) pour commander l'intensité de la lumière d'éclairement provenant de ladite source lumineuse en appliquant le signal de sortie desdits moyes d'intégration à la source d'énergie de la lampe de ladite source lumineuse.

# FIG.1

# FIG.2

# FIG.3

# FIG.4

16R
16
16B
16G

# FIG.6

23
16
19
18
10
32
13    12    14    36    33    17

# FIG.5

# FIG.7

# FIG.8